# EUROPEAN PATENT APPLICATION

(11) **EP 3 750 523 A1**
(43) Date of publication of application: **16.12.2020**
(21) Application number: 19179950.1
(22) Date of filing: 13.06.2019
(51) Int. Cl.: A61K 9/00, A61K 9/51, A61K 47/34

(54) **ACTIVE SUBSTANCE DELIVERY SYSTEM**

(71) Applicant: CAPNOMED GmbH, 78658 Zimmern (DE); Universität des Saarlandes, 66123 Saarbrücken (DE)
(72) Inventor: Reymond, Marc A., 72076 Tübingen (DE); Schneider, Marc, 66125 Saarbrücken (DE); Linder, Alexandra, 69123 Heidelberg (DE); Sahoo, Ranjita, 47574 Goch (DE)
(74) Representative: Eisenführ Speiser

(57) **Abstract**

The present invention relates to an active substance delivery system, preferably an anti-cancer agent delivery system, comprising one or more active substances included in nanoparticles and a method for producing such a delivery system.

## Description

The present invention relates to an active substance delivery system, preferably an anticancer agent delivery system and a method for producing such a delivery system.

Cancer is the second leading cause of death globally, and is responsible for an estimated 9.6 million deaths in 2018. Globally, about 1 in 6 deaths is due to cancer. Among the most common cancers are lung cancer (2.09 million cases, 1.76 million deaths in 2018), breast cancer (2.09 million cases, 627,000 deaths in 2018), colorectal cancer (1.80 million cases, 862,000 deaths in 2018), prostate cancer (1.28 million cases) and stomach cancer (1.03 million cases, 783,000 deaths in 2018).

Metastases are new pathological sites spread by a cancerous (e.g. malignant) tumour. Malignant tumours are capable to invade into adjacent and distant tissue, e.g. by invasion into the blood circulation, followed by invasion into another site, where another tumour (i.e. a metastasis) may grow. Likewise, such tumours may spread along various tissues, which complicates especially a surgical removal of the tumour.

The cancer site as used herein is the part or cavity of the body, i.e. the respective tissue or part of a tissue, where cancer cells located. Cavities also include hollow organs. Often, hollow organs such as the esophagus, the stomach, the urinary bladder or other organs, e.g. containing a virtual space and lined with an epithelium, may serve as cancer site.

Particularly in view of cancer sites, the abdominal cavity and the pleural cavities are important regions of the body. The abdomen stretches from the thorax at the thoracic diaphragm to the pelvis at the pelvic brim. The region occupied by the abdomen is termed the abdominal cavity and contains many organs such as the stomach, the small intestine and the colon, the liver, the gall bladder and the pancreas. The abdominal cavity and the pleural cavities are coated by an extensive membrane, called the peritoneum or, respectively, the pleura, which covers the abdominal wall and the pelvic walls (parietal peritoneum) as well as the included organs (visceral peritoneum) or, respectively the inner surface of the thoracic cavity (parietal pleura) as well as the included organs (visceral pleura).

A special form of metastases are abdominal, particularly peritoneal metastases. As the cancer cells are attached to the outside of several organs and tissues but reach into the area of the peritoneum (and are thus classified as abdominal or particularly peritoneal or, respectively, pleural), these metastases are more difficult to reach with medication via the blood circulation. Cancer cells may therefore "escape" into the peritoneum and/or peritoneal cavity. Still, in many cases, these cancer sites are covered by the peritoneum, i.e. are positioned between the respective organ or tissue and the peritoneum or the pleura.

The terms "peritoneal metastasis" and "peritoneal cancer" as used herein may be understood as synonyms. Both terms comprise cancer cells of a primary or a secondary site of the host's body.

The terms "pleural metastasis" and "pleural cancer" as used herein may be understood as synonyms. Both terms comprise cancer cells of a primary or a secondary site of the host's body.

The treatment options for cancer strongly depend on several factors such as the involved organs, the cause of cancer and further complicating factors as well as the age and health status of the patient.

Although many limitations, for various cancer types, a systemic chemotherapy is applied as a standard of care. However, several tissues have a poor vascularisation and can thus be poorly reached by a systemically applied chemotherapy. The therapy thus results in a poor performance. As a counteractive measure, higher doses and/or higher volumes of the chemotherapeutic agent(s) could be applied transport higher amounts of the chemotherapeutic agent into the poorly vascularised tissues. However, increasing concentrations also increase the risk of dangerous adverse effects, renal toxicity, neurotoxicity or cardiac toxicity.

As an improvement to systemic chemotherapy, local chemotherapy can be applied to patients with tumour sites in poorly vascularized tissues. The delivery of the chemotherapeutically active substance is thereby localised to a desired tissue or region.

Such treatment options have recently emerged particularly for cancer sites in the poorly vascularized peritoneal cavity or pleural cavities:
Possible treatment options for peritoneal metastasis are e.g. Hyperthermic Intraperitoneal Chemotherapy (HIPEC) or Pressurized Intraperitoneal Aerosol Chemotherapy (PIPAC). Possible treatment options for pleural metastasis is Pressurized Intrathoracic Aerosol Chemotherapy (PITAC). In some cases, electro-precipitation may also be an option (as described for example in Kakchekeeva et al., In Vivo Feasibility of Electrostatic Precipitation as an Adjunct to Pressurized Intraperitoneal Aerosol Chemotherapy (ePIPAC), Ann Surg Oncol. 2016 Dec;23(Suppl 5):592-598 or in Reymond et al., Electrostatic precipitation Pressurized IntraPeritoneal Aerosol Chemotherapy (ePIPAC): first in-human application, Pleura Peritoneum, 2016 Jun 1;1(2):109-116).

Hyperthermic Intraperitoneal Chemotherapy (HIPEC) is a treatment usually performed after surgery when all visible cancer sites in the abdomen are surgically removed. A liquid heated chemotherapy is applied to the peritoneal cavity, bathing all reachable organs and their surfaces. With this treatment, any remaining cancer cells shall be killed. However, this treatment is a time-consuming process with higher mortality rates.

Pressurized Intraperitoneal Aerosol Chemotherapy (PIPAC) as well as Pressurized Intrathoracic Aerosol Chemotherapy (PITAC) are palliative treatments of peritoneal or, respectively, pleural metastasis. PIPAC is a method for applying chemotherapeutic drugs, in form of an aerosol, under pressure into the abdominal cavity. The aerosol is usually applied within the closed abdominal or, respectively, thoracic cavity, i.e. during a laparoscopy or, respectively, thoracoscopy. PIPAC and PITAC are significantly time saving therapies compared to other available techniques like HIPEC.

In many cases, however, sole chemotherapy is not sufficient for treating cancer and a surgery, in which the cancer cells are removed, becomes necessary or even the only remaining option. Surgeries generally provide risks to patients such as (internal) bleedings, infections and stress for the cardiovascular system during anaesthesia. Nevertheless, surgeries are usually necessary if performed. However, to prevent the risks involved, unnecessary surgeries should be avoided.

A drawback of the surgical removal of cancer cells is that in several cases, some cancer cells or cancer cell clusters remain in the body, as they were too small to be seen or detected during surgery. These cancer cells or cancer cells clusters usually tend to be very aggressive, i.e. are rapidly growing and likely to spread in form of metastases.

Furthermore, healing includes the rapid growth of tissue in proximity of the wound, which is usually triggered or accompanied by a mixture of endogenous growth stimulants. Such stimulants may also promote growth of the remaining cancer cells or cancer cell clusters.

As the body is weakened by performing the surgery, a systemic chemotherapy to kill the remaining cancer cells or cancer cell clusters is not an option. Also, local chemotherapy often cannot be performed, as the substances themselves usually interfere with the healing process or as the local application of the chemotherapeutic agent is performed under pressure (e.g. in case of PIPAC or PITAC) or other (physical) interactions with the tissue affected by surgery.

Moreover, even if the chemotherapeutic agents reach their destination, their concentration must be low enough to ensure proper wound healing but at the same time high enough to fight the remaining cancer cells or cancer cell clusters. Thus, their concentration needs to be tightly regulated overtime (concentrations may and should be increased with increasing healing progress) and be analysed to intervene if it becomes necessary.

Therefore, the primary object of the present invention is to provide new and improved treatment measures for treating cancer or in connection with treating cancer, particularly shortly after a surgery for removing a tumour, preferably to provide new and improved delivery systems to be used in the treatment of cancer, wherein one or more, particularly preferably all, of the above-mentioned drawbacks can be avoided or overcome.

The primary object of the present invention is achieved by an active substance delivery system comprising or consisting of
a) nanoparticles comprising or consisting of
   a1) 0.05 to 15 wt.-%, preferably 0.075 to 12 wt.-%, based on the total weight of the nanoparticles, of one, two, three or more active substances, wherein the, one, two, three, several or all active substances are selected from the group consisting of cancer chemotherapeutic agents, immunomodulatory drugs, antibodies, viruses, adjuvants such as vitamins, enzymes, and combinations thereof, and
   a2) 75 to 99.9 wt.-%, preferably 80 to 95 wt.-%, based on the total weight of the nanoparticles, of poly(lactic-co-glycolic acid) (PLGA), and
   a3) optionally: 0.5 to 20 wt.-%, preferably 0.5 to 15 wt.-%, particularly preferably 0.5 to 10 wt.-%, especially preferably 0.5 to 7 wt.-% based on the total weight of the nanoparticles, of one, two, three or more surface coating material(s), wherein the, one, two, three, several or all surface coating material(s) is/are selected from the group consisting of chitosan, carboxymethyl chitosan, cellulose, starch, polyethylene glycols (PEG), polyvinyl alcohols, polymers or block co-polymers (such as polyacrylic acid, poly-L-lysine, polytheylenimine or PDMAAm), dextran, albumin, surfactants and pullulan,
   preferably wherein the weight ratio of a2) to a1) is in a range of from 2 : 1 to 150 : 1,
   and wherein the average size of the nanoparticles is in a range of from 100 to 350, preferably in a range of from 120 to 250 nm, particularly preferably in a range of from 140 to 230 nm,
b) optionally: one or more further pharmaceutically acceptable components,
for use in the treatment of cancer in a human subject, wherein the treatment comprises one or more applications of the delivery system to the human subject, preferably by direct introduction into the abdomen and/or thorax.

It is an advantage of the active substance delivery system according to the invention or, respectively, the nanoparticles according to the invention that the release of the active substance(s) from the nanoparticles can be delayed. Own experiments showed that the active substance delivery system according to the invention or, respectively, the nanoparticles according to the invention show release characteristics that are particularly useful for delaying the release of the active substance(s) from the nanoparticles, particularly with regard to a release to the abdomen or thorax of a subject.

Preferably, the subject is a human, preferably a human suffering from cancer, such as a cancer of any body cavity, preferably a cancer type selected from the group consisting of gastrointestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

The treatment as described herein can comprise or consist of a parenteral administration of the delivery system, preferably an intraperitoneal and/or intrathoracic administration of the delivery system.

The treatment as described herein preferably comprises an administration of the delivery system before, during or after surgery, preferably by systemic application, preferably an oral or intravenous application, or by local application, preferably an intraperitoneal and/or intrathoracic application.

The treatment of cancer in a human subject can be any kind of treatment of cancer. Particularly, the treatment is directed to cancer in a body cavity. Preferably, the treatment is directed to a cancer type selected from the group consisting of gastrointestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin. The treatment as described herein can be directed to reducing tumour size and the amount of tumour sites. Tumour sites includes the primary tumour as well as metastases and further tumour reproductions. The treatment can also be directed to prevent or delay cancer recurrence after tumour removal or partial tumour removal, preferably after a surgical tumour removal.

It is preferred that the treatment comprises one, two, three or more administration(s) of the delivery system, particularly preferably one administration of the delivery system.

Preferably, the, one, two, three or all administration(s) of the delivery system is/are performed with an assisting tool selected from the group consisting of microneedles, spray devices, angio-injectors, or any combination thereof, preferably with a nebulizer, spraying gun or spray catheter, particularly preferably the assisting tool is a laparoscopic nebulizer, especially preferably the laparoscopic nebulizer known as Capnopen, preferably as described in US 9,511,197 B2.

Preferably, the, one, two, several or all chemotherapeutic agents used within the scope of the present invention is/are selected from the group consisting of chemotherapeutic agents used against a cancer type selected from the group consisting of gastrointestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin.

Typically, and preferably such chemotherapeutic agents are selected from the group consisting of cisplatin, doxorubicin, paclitaxel and oxaliplatin and a combination thereof.

The amount of the or, respectively, each active substance, preferably chemotherapeutic agent, is preferably contained in a range of from 120 mg to 2500 mg, preferably of from 150 mg to 2000 mg, particularly preferably of from 150 mg to 1500 mg, especially preferably of from 150 mg to 800 mg in the delivery system or, respectively, the nanoparticles, preferably this indication refers to the total amount administered during one application, preferably as a powder or suspended or redispersed in physiological solutions like saline. Additionally or alternatively, the amount of the or, respectively, each active substance, preferably chemotherapeutic agent, is preferably contained in a range of from 30 mg/m² body surface to 250 mg/m² body surface, preferably of from 40 mg/m² body surface to 200 mg/m² body surface, particularly preferably of from 50 mg/m² body surface to 180 mg/m² body surface, especially preferably of from 50 mg/m² body surface to 150 mg/m² body surface in the delivery system or, respectively, the nanoparticles, wherein this indication refers to the total amount administered during one application, preferably as a powder or suspended or redispersed in physiological solutions like saline. The amounts of the active substance may, however, vary with the respective active substance(s) and/or type of active substance(s). Thus, the amount of the or, respectively, each active substance, preferably chemotherapeutic agent, as described, may e.g. also be contained in a range of from 50 mg/m² body surface to 70 mg/m² body surface.

The term "body surface" as used herein refers to the body surface of the human subject receiving the active substance delivery system according to the invention or, respectively, the nanoparticles according to the invention. The body surface can be calculated from the size of the human subject and its body weight and is well known to a person skilled in the art.

Alternatively, or in addition to the chemotherapeutic agent(s), component a1) can include one or more immunomodulatory agent(s), preferably such as curcumin, lutein, hesperidin, apigenin, hesperetin, ajoene, arctigenin, β-carotene, epigallocatechin-3-gallate, glabridin or quinic acid or any other suitable chemical entity which exerts immunomodulatory effects.

It is preferred that component a1) comprises one chemotherapeutic agent but no further agent.

It is also preferred that component a1) comprises two or more chemotherapeutic agents, but no further agent.

Moreover, it is preferred that component a1) comprises one chemotherapeutic agent and one immunomodulatory agent.

It is also preferred that component a1) comprises one chemotherapeutic agent and two or more immunomodulatory agents.

Furthermore, it is preferred that component a1) comprises two or more chemotherapeutic agents and one immunomodulatory agent.

Further, it is also preferred that component a1) comprises two or more chemotherapeutic agents and two or more immunomodulatory agents.

It is preferred that the weight ratio of components a2) and a1) is in a range of from 2 : 1 to 150 : 1, preferably in a range of from 5 : 1 to 100 : 1, preferably in a range of from 10 : 1 to 150 : 1, particularly preferably in a range of from 10 : 1 to 100 : 1, especially preferably in a range of from 20 : 1 to 50 : 1. If component a1) consists of more than one substance, the amount by weight of component a1) refers to the sum of the weights of these substances.

Preferably, the one or more further pharmaceutically acceptable component(s) is/are selected from the group consisting of carriers, polymers, surfactants, stabilizers, wetting agents, emulsifiers, antioxidants, pH influencing agents, disintegrants, recrystallization agents, fluxing agents, preservatives, solvents, salts, fillers, binders, foamers, defoamers, lubricants, adsorbents, for adjusting the osmotic pressure and buffers.

It is particularly preferred if the active substance delivery system as described herein or the nanoparticles as described herein can be administered with an assisting tool selected from the group consisting of microneedles, spray devices, angio-injectors, or any combination thereof, preferably with a nebulizer, spraying gun or spray catheter, particularly preferably the assisting tool is a laparoscopic nebulizer, especially preferably the laparoscopic nebulizer known as Capnopen, preferably as described in US 9,511,197 B2.

Furthermore, the present invention relates to a method for producing an active substance delivery system preferably an active substance delivery system according to the invention or, respectively, the nanoparticles according to the invention or the nanoparticles as described herein comprising or consisting of the following steps:
i) providing PLGA and dissolving PLGA in a solvent,
ii) providing one, two, three or more active substances and dissolving the one, two, three or more active substances in a solvent,
iii) mixing the solutions obtained in step i) and ii),
iv) providing a polyvinyl alcohol (PVA) solution, preferably a solution containing 0.1 to 5 wt.-%, particularly preferably 0.2 to 3 wt.-% of PVA, based on the total amount of the solution,
v) adding the mixture obtained in step iii) to the solution obtained in step iv) to produce nanoparticles,
vi) optionally: evaporating the solvent, one of or the solvents used in step i) and ii),
vii) purifying the nanoparticles and subsequently dispersing the nanoparticles in a dispersant, preferably in a pharmaceutically acceptable solvent,
viii) providing one, two, three or more surface coating material(s) and coating the nanoparticles therewith, and
ix) optionally: providing and adding one or more further pharmaceutically acceptable components to the nanoparticles
wherein in step v) the amounts of the mixture and of the solution are selected such that a ratio of a least 1 ml of solution per 5 mg of the summed weight of PLGA and the active substance(s) is achieved, and
wherein the addition in step v) is performed by injection, preferably constant injection, preferably at a rate in a range of from 0.1 to 1.75, preferably in a range of from 0.25 to 1.5 ml/min, particularly preferably at a rate in a range of from 0.35 to 1.25 ml/min.

The term "solvent" as used herein can be a single substance but also a mixture of substances. Thus, "solvent" may also encompass several different substances, e.g. several different single solvents.

Preferably, the solvent used in step i) and/or the solvent used in step ii) comprises or consists of one or more substances selected from the group consisting of acetone, ethyl acetate, chlorinated solvents, DMSO, methylated solvents, tetrahydrofuran, halogenated hydrocarbons such as chloroform and dichloromethane, dioxanes and acetonitrile.

It is further preferred that additionally or alternatively, the solvent used in step iv) for providing a PVA solution comprises or consists of one or more substances selected from the group consisting of water, ethanol and saline.

Additionally, or alternatively, the dispersant used in step vii) comprises or consists of one or more substances selected from the group consisting of saline, glucose, surfactants, pH modifiers and water.

It is particularly preferred if the, one or all active substance(s) of component a1) of the active substance delivery system according to the invention or of nanoparticles as described herein is/are (a)
i) chemotherapeutic agent(s), preferably selected from the group consisting of chemotherapeutic agents used against a cancer type selected from the group consisting of gastrointestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin, particularly preferably selected from the group consisting of paclitaxel, cisplatin, oxaliplatin and doxorubicin,
   and/or
ii) immunomodulatory drug(s), preferably selected from the group consisting of curcumin, lutein, hesperidin, apigenin, hesperetin, ajoene, arctigenin, β-carotene, epigallocatechin-3-gallate, glabridin or quinic acid or any other suitable chemical entity which exerts immunomodulatory effects.

Additionally, or alternatively, the one, two, three or more active substances can comprise or consist of one or more antibodies, one or more adjuvants, such as vitamins; and enzymes.

Also preferred is that additionally or alternatively, the amount of the or, respectively, each active substance, preferably chemotherapeutic agent, is preferably contained in a range of from 120 mg to 2500 mg, preferably of from 150 mg to 2000 mg, particularly preferably of from 150 mg to 1500 mg, especially preferably of from 150 mg to 800 mg in the delivery system or, respectively, the nanoparticles, preferably this indication refers to the total amount administered during one application, preferably as a powder or suspended in physiological solutions like saline.

Also preferred is that additionally or alternatively, the amount of the or, respectively, each active substance, preferably chemotherapeutic agent, is preferably contained in a range of from 30 mg/m² body surface to 250 mg/m² body surface, preferably of from 40 mg/m² body surface to 200 mg/m² body surface, particularly preferably of from 50 mg/m² body surface to 180 mg/m² body surface, especially preferably of from 50 mg/m² body surface to 150 mg/m² body surface in the delivery system or, respectively, the nanoparticles, wherein this indication refers to the total amount administered during one application, preferably as a powder or suspended or redispersed in physiological solutions like saline. The amounts of the active substance may, however, vary with the respective active substance(s) and/or type of active substance(s). Thus, the amount of the or, respectively, each active substance, preferably chemotherapeutic agent, as described, may e.g. also be contained in a range of from 50 mg/m² body surface to 70 mg/m² body surface.

Particularly preferably, the active substance delivery system as described herein or the nanoparticles as described herein and/or the active substance delivery system or the nanoparticles as produced by a method as described herein comprise two active substances, especially preferably a chemotherapeutic agent, such as paclitaxel, cisplatin, oxaliplatin or doxorubicin, and an immunomodulatory drug, such as curcumin, lutein, hesperidin, apigenin, hesperetin, ajoene, arctigenin, β-carotene, epigallocatechin-3-gallate, glabridin or quinic acid or any other suitable chemical entity which exerts immunomodulatory effects.

It is preferred that the weight ratio of components a2) and a1) is in a range of from 2 : 1 to 150 : 1, preferably in a range of from 5 : 1 to 100 : 1, preferably in a range of from 10 : 1 to 150 : 1, particularly preferably in a range of from 10 : 1 to 100 : 1, especially preferably in a range of from 20 : 1 to 50 : 1. If component a1) consists of more than one substance, the amount by weight of component a1) refers to the sum of the weights of these substances.

Further preferred is if the, one, two, three, several or all surface coating material(s) is/are selected from the group consisting of chitosan, carboxymethyl chitosan, cellulose, starch, polyethylene glycols (PEG), polyvinyl alcohols, polymers or block co-polymers (such as polyacrylic acid, poly-L-lysine, polytheylenimine or PDMAAm), dextran, albumin, surfactants and pullulan.

The coating is preferably performed by a one pot synthesis or crosslinking adding a hydrophilic compound in the polar medium. Using such an approach and a polyelectrolyte of the respective charge the overall surface potential of the particles can be adapted. This might be of use for improved interaction with cell membranes, improved uptake but also for modified deposition behaviour depending on the setup.

As a second approach the consecutive coating might be used. Preparation of loaded particles and then the addition to the aqueous polymeric solution for adsorption and surface coating may also be an option.

Further preferred is if additionally, or alternatively, the total amount of the surface coating material(s) is in a range of from 1 to 20 wt.-%, preferably in a range of from 3 to 15 wt.-%, particularly preferably in a range of from 5 to 10 wt.-%, based on the total weight of the nanoparticles.

Preferably, the one or more further pharmaceutically acceptable component(s) is/are selected from the group consisting of carriers, polymers, surfactants, stabilizers, wetting agents, emulsifiers, antioxidants, pH influencing agents, disintegrants, recrystallization agents, fluxing agents, preservatives, solvents, salts, fillers, binders, foamers, defoamers, lubricants, adsorbents, for adjusting the osmotic pressure and buffers.

It is also preferred if the addition of the mixture, obtained in step iii), in step v) of the described method is performed by injection, i.e. by slowly adding the mixture to the polyvinyl alcohol (PVA) solution by using a syringe pump (equipped with a syringe and a cannula). Preferably, the injection is a constant injection, especially preferably at a rate in a range of from 0.1 to 1.75, preferably in a range of from 0.25 to 1.5 ml/min, particularly preferably at a rate in a range of from 0.35 to 1.25 ml/min. The controlled amount of injected material offers a fast diffusion and thus precipitation process. The quality of the nanoparticles in terms of average size but also the size distribution can thus be improved by using such a technique.

The term constant injection as used herein describes a constant injection rate after an initial phase and before an end phase. In the initial phase and in the and end phase, the injection rate is typically lower than the intended constant injection rate and is increased to achieve the intended constant injection rate or, respectively, decreases as the material to be injected fades and may no longer uphold the intended constant injection rate. In the initial phase, while the constant injection rate is adjusted, the injection rate may also exceed the intended constant injection rate. It is preferred that 75 wt.-% of the material to be injected, preferably 80 wt.-%, particularly preferably 90 wt.-% or 95 wt.-%, is injected by means of constant injection as described herein.

Typically, the particle size of the nanoparticles is measured by dynamic light scattering techniques and electron microscopy or scanning probe microscopy. The size can also be determined by fractionation with disc centrifugation and flow techniques. Further methods are known to a person skilled in the art and may also be applied.

The invention is further related to an active substance delivery system for use as described herein and produced or producible by the method as described herein. What was said herein with regard to the delivery system may also apply to the delivery system produced or producible with a method as described herein. What was said herein with regard to the method for producing an active substance delivery system may also apply to a delivery device produced with such a method, where applicable.

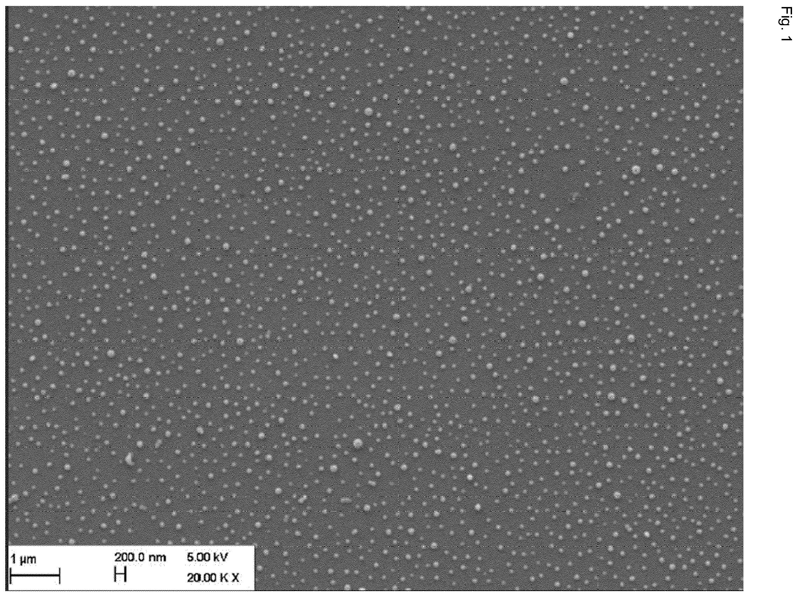

Figure 1 is a picture of exemplary nanoparticles as described herein, consisting of PLGA and loaded with paclitaxel, obtained with a scanning electron microscope (SEM).

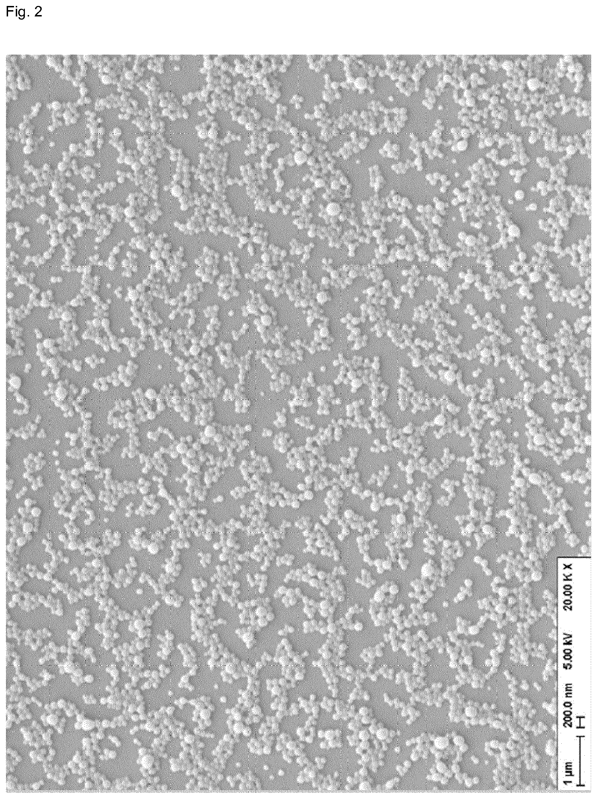

Figure 2 is a picture of exemplary nanoparticles as described herein, consisting of PLGA and loaded with curcumin, obtained with a scanning electron microscope (SEM).

Preferred embodiments and further aspects of the present invention also emerge from the attached patent claims and the following examples, wherein the present invention is not limited to these examples.

### Examples:

### Example 1: Chemotherapeutic agent

| Ingredient | Amount [mg] | | |
|---|---|---|---|
| | A | B | C |
| PLGA | 24.75 | 24.50 | 24.75 |
| Paclitaxel | 0.25 | 0.50 | 0.25 |
| Acetone | 1.375 | 1.250 | 4.875 |
| Acetonitrile | 0.125 | 0.250 | 0.125 |

PLGA was provided and dissolved in acetone. Additionally, paclitaxel was provided separately and dissolved in acetonitrile. Both solutions were mixed.

10 ml of a 2 % polyvinyl alcohol (PVA) solution were prepared, wherein the polyvinyl alcohol was dissolved in water.

The mixture obtained above was added to the PVA solution by injection with a syringe pump with a constant rate of 0.5 ml/min. Upon addition, nanoparticles were produced.

The nanoparticles were further purified by 2 x 20 min of centrifugation at 10.000 x g and at 20 °C. The supernatant was removed and the pelleted nanoparticles were redispersed in 5 ml of water and 0.25 g of glucose.

The nanoparticles had an average size (diameter) of 146 to 217 nm.

### Example 2: Immunomodulatory drug

| Ingredient | Amount [mg] | | | | |
|---|---|---|---|---|---|
| | D | E | F | G | H |
| PLGA | 39.6 | 39.6 | 29.5 | 95 | 99 |
| Curcumin | 0.4 | 0.4 | 0.5 | 5 | 1 |
| Acetone | - | - | 2 | - | 4 |
| Acetonitrile | 4 | 3 | - | 6 | - |
| Chitosan | - | - | - | - | 6 |

| | | | | | |
|---|---|---|---|---|---|
| PLGA was provided and dissolved in acetone or acetonitrile. Additionally, curcumin was provided separately. | | | | | |

For examples 2D to 2F, 20 ml of a 2 % polyvinyl alcohol (PVA) solution were prepared, wherein the polyvinyl alcohol was dissolved in water. Furthermore, for example 2H, chitosan was additionally added to the PVA solution for coating the nanoparticles.

For examples 2G and 2H, 40 ml of a 2 % polyvinyl alcohol (PVA) solution were prepared, wherein the polyvinyl alcohol was dissolved in water.

The mixture obtained above was added to the PVA solution by injection with a syringe pump. Examples 2D, 2F, 2G and 2H were added at a constant rate of 0.5 ml/min. Example 2E was added at a constant rate of 1 ml/min. Upon addition, nanoparticles were produced. Furthermore, the nanoparticles were coated with chitosan.

The nanoparticles were further purified by 3 x 10 min of centrifugation (Examples 2D to 2F), by 3 x 15 min of centrifugation (Example 2G) or by 3 x 20 min of centrifugation (Example 2H) at 10.000 x g (Examples 2D and 2E) or at 15.000 x g (Examples 2F to 2H) and at 20 °C. The supernatant was removed and the pelleted nanoparticles were redispersed in 20 ml of water.

The nanoparticles of examples 2D, 2F, 2G and 2H had an average size (diameter) of 189 to 231 nm. The included nanoparticles of examples 2E had an average size (diameter) of 221 to 233 nm.

### Example 3: Chemotherapeutic agent and immunomodulatory drug

| Ingredient | Amount [mg] | | |
|---|---|---|---|
| | I | J | K |
| PLGA | 49,00 | 49.25 | 49.45 |
| Paclitaxel | 0.50 | 0.50 | 0.50 |
| Curcumin | 0.50 | 0.25 | 0.05 |
| Acetone | 4.975 | 4.975 | 4.975 |
| Acetonitrile | 0.025 | 0.025 | 0.025 |

| | | | |
|---|---|---|---|
| PLGA was provided and dissolved in acetone. Additionally, paclitaxel and curcumin were provided separately and dissolved in acetonitrile. | | | |

10 ml of a 2 % polyvinyl alcohol (PVA) solution were prepared, wherein the polyvinyl alcohol was dissolved in water.

The mixture obtained above was added to the PVA solution by injection with a syringe pump at a constant rate of 0.5 ml/min to produce nanoparticles.

The nanoparticles were further purified by 3 x 15 min of centrifugation at 15.000 x g and at 20 °C. The supernatant was removed and the pelleted nanoparticles were redispersed in 20 ml of water.

The nanoparticles had an average size (diameter) of 177 to 207 nm.

### Example 4: Spraying of preheated unloaded nanoparticles

The following experiment was performed to examine an effect of preheating and spraying on nanoparticle properties.

Therefore, several batches of unloaded nanoparticles were prepared:
For each batch of unloaded nanoparticles 500 mg PLGA was provided and dissolved in 30 mL acetonitrile.

200 mL of a 2 % polyvinyl alcohol (PVA) solution were prepared, wherein the polyvinyl alcohol was dissolved in water.

The polymer solutions obtained above were added to the PVA solution by injection with a syringe pump at a constant rate of 0.5 ml/min to produce nanoparticles.

Optionally: The solvents are evaporated overnight.

The nanoparticles were further purified by 3 x 15 min of centrifugation at 15.000 x g and at 20 °C. The supernatant was removed, and the pelleted nanoparticles were redispersed in 2 ml of water. All batches were pooled.

The nanoparticles had a size (diameter) of 185 nm.

Nanoparticle concentrations of 1, 0.75 and 5% were prepared as 30 ml triplicates and were preheated to 30 degrees while stirring. Then the preheated particles were sprayed with a flow rate of 0.5 ml/s through a nebulizer onto water (MilliQ) in a distance of 5 cm. Spraying was performed with a heated cuff at room temperature (22 °C).

Zeta sizer measurements of the heated as well as the heated and sprayed samples as described showed no significant change in particle properties.

### Example 5: Spraying of unloaded and curcumin-loaded nanoparticles

The following experiment was performed to examine an effect of spraying on properties of unloaded and curcumin-loaded nanoparticles.

Therefore, several batches of unloaded and curcumin loaded nanoparticles were prepared:
For each batch curcumin loaded nanoparticles 500 mg PLGA was provided and dissolved in 29.5 ml acetonitrile. Additionally, 5 mg per batch curcumin was provided separately and dissolved in 0.5 ml acetonitrile and mixed to the PLGA-acetonitrile mixture. For each batch of unloaded nanoparticles 500 mg PLGA were provided and dissolved in 30 ml acetonitrile.

200 ml of a 2 % polyvinyl alcohol (PVA) solution were prepared, wherein the polyvinyl alcohol was dissolved in water.

The polymer solutions obtained above was added to the PVA solution by injection with a syringe pump at a constant rate of 0.5 ml/min to produce nanoparticles.

Optionally: The solvents are evaporated overnight.

The nanoparticles were further purified by 3 x 15 min of centrifugation at 15.000 x g and at 20 °C. The supernatant was removed, and the pelleted nanoparticles were redispersed in 2 ml of water. All batches were pooled.

The nanoparticles had a size (diameter) of 177 nm (unloaded) and 184 nm (curcumin loaded).

Nanoparticle concentrations of 1, 0.75 and 5% were prepared as 30 ml triplicates and were sprayed with a flow rate of 0.5 ml/s through a nebulizer onto water (MilliQ) in a distance of 5 cm. Spraying was performed at room temperature (22 °C).

Zeta sizer measurements of the sprayed samples as described showed no significant change in particle properties by spraying.

## Claims

1. Active substance delivery system comprising or consisting of
a) nanoparticles comprising or consisting of
a1) 0.05 to 15 wt.-%, preferably 0.075 to 12 wt.-%, based on the total weight of the nanoparticles, of one, two, three or more active substances, wherein the, one, two, three, several or all active substances are selected from the group consisting of cancer chemotherapeutic agents, immunomodulatory drugs, antibodies, viruses, adjuvants such as vitamins and enzymes, and combinations thereof, and
a2) 75 to 99.9 wt.-%, preferably 80 to 95 wt.-%, based on the total weight of the nanoparticles, of poly(lactic-co-glycolic acid) (PLGA), and
a3) optionally: 0.5 to 20 wt.-%, preferably 0.5 to 15 wt.-%, particularly preferably 0.5 to 10 wt.-%, especially preferably 0.5 to 7 wt.-% based on the total weight of the nanoparticles, of one, two, three or more surface coating material(s), wherein the, one, two, three, several or all surface coating material(s) is/are selected from the group consisting of chitosan, carboxymethyl chitosan, cellulose, starch, polyethylene glycols (PEG), polyvinyl alcohols, polymers or block co-polymers (such as polyacrylic acid, poly-L-lysine, polytheylenimine or PDMAAm), dextran, albumin, surfactants and pullulan
and wherein the average size of the nanoparticles is in a range of from 100 to 350, preferably in a range of from 120 to 250 nm, particularly preferably in a range of from 140 to 230 nm
b) optionally: one or more further pharmaceutically acceptable components,
for use in the treatment of cancer in a human subject, wherein the treatment comprises one or more applications of the delivery system to the human subject, preferably by direct introduction into the abdomen and/or thorax.

2. Active substance delivery system for use according to claim 1, wherein the weight ratio of a2) to a1) is in a range of from 2 : 1 to 150 : 1.

3. Active substance delivery system for use according to one of the preceding claims, wherein the, one or more further pharmaceutically acceptable components is/are selected from the group consisting of carriers, polymers, surfactants, stabilizers, wetting agents, emulsifiers, antioxidants, pH influencing agents, disintegrants, recrystallization agents, fluxing agents, preservatives, solvents, salts fillers, binders, foamers, defoamers, lubricants, adsorbents for adjusting the osmotic pressure and buffers.

4. Active substance delivery system for use according to one of the preceding claims, wherein the delivery system or the nanoparticles are preparable or prepared by a method comprising or consisting of the following steps:
i) providing PLGA and dissolving PLGA in a solvent,
ii) providing one, two, three or more active substances and dissolving the one, two, three or more active substances in a solvent,
iii) mixing the solutions obtained in step i) and ii),
iv) providing a polyvinyl alcohol (PVA) solution, preferably a solution containing 0.1 to 5 wt.-%, particularly preferably 0.2 to 3 wt.-% of PVA, based on the total amount of the solution,
v) adding the mixture obtained in step iii) to the solution obtained in step iv) to produce nanoparticles,
vi) optionally: evaporating the solvent, one of or the solvents used in step i) and ii),
vii) purifying the nanoparticles and subsequently dispersing the nanoparticles in a dispersant, preferably in a pharmaceutically acceptable solvent,
viii) providing one, two, three or more surface coating material(s) and coating the nanoparticles therewith, and
ix) optionally: providing and adding one or more further pharmaceutically acceptable components to the nanoparticles
wherein in step v) the amounts of the mixture and of the solution are selected such that a ratio of a least 1 ml of solution per 5 mg of the summed weight of PLGA and the active substance(s) is achieved, and
wherein the addition in step v) is performed by injection, preferably constant injection, preferably at a rate in a range of from 0.1 to 1.75, preferably in a range of from 0.25 to 1.5 ml/min, particularly preferably at a rate in a range of from 0.35 to 1.25 ml/min.

5. Active substance delivery system for use according to one of the preceding claims, wherein the solvent used in step i) and/or the solvent used in step ii) comprises or consists of one or more substances selected from the group consisting of acetone ethyl acetate, chlorinated solvents, DMSO, methylated solvents, tetrahydrofuran, halogenated hydrocarbons such as chloroform and dichloromethane, dioxanes and acetonitrile,
and/or
wherein the solvent used in step iv) for providing a PVA solution comprises or consists of one or more substances selected from the group consisting of water, ethanol and saline,
and/or
wherein the dispersant used in step vii) comprises or consists of one or more substances selected from the group consisting of saline, glucose, surfactants, pH modifiers and water.

6. Active substance delivery system for use according to one of the preceding claims, wherein the, one or all active substance(s) is/are (a)
i) chemotherapeutic agent(s), preferably selected from the group consisting of chemotherapeutic agents used against a cancer type selected from the group consisting of gastrointestinal cancer, particularly gastric cancer, colorectal cancer, hepatobiliary or pancreatic cancer, appendix cancer, esophageal cancer, hepatocellular carcinoma; particularly primary peritoneal cancer, ovarian cancer, endometrial cancer; prostate cancer, leukaemia, lymphoma, soft-tissue sarcoma, multiple myeloma, bladder cancer, lung cancer, thyroid cancer, Kaposi's sarcoma and tumours of embryonal origin, particularly preferably selected from the group consisting of paclitaxel, cisplatin, oxaliplatin and doxorubicin,
and/or
ii) immunomodulatory drug(s), preferably selected from the group consisting of curcumin, lutein, hesperidin, apigenin, hesperetin, ajoene, arctigenin, β-carotene, epigallocatechin-3-gallate, glabridin or quinic acid.

7. Active substance delivery system for use according to one of the preceding claims, wherein the treatment is directed to a cancer type selected from the group consisting of gastrointestinal cancer, gynaecological cancer, prostate cancer, peritoneal metastasis, leukaemia, lymphoma, soft-tissue sarcoma, multiple myeloma, breast cancer, bladder cancer, lung cancer, thyroid cancer, Kaposi's sarcoma, particularly preferably ovarian cancer, peritoneal metastasis colorectal cancer or stomach cancer.

8. Active substance delivery system for use according to one of the preceding claims, wherein the, one or all administration(s) of the active substance delivery system is/are performed with an assisting tool selected from the group consisting of microneedles, spray devices, angio-injectors, or any combination thereof, preferably with a nebulizer, spraying gun or spray catheter.

9. Active substance delivery system for use according to one of the preceding claims, wherein in the treatment, the total amount of active substance(s), preferably chemotherapeutic agent(s), administered during one application is in a range of from
120 mg to 2500 mg, preferably of from 150 mg to 2000 mg, particularly preferably of from 150 mg to 1500 mg, especially preferably of from 150 mg to 800 mg
and/or
30 mg/m² body surface to 250 mg/m² body surface, preferably of from 40 mg/m² body surface to 200 mg/m² body surface, particularly preferably of from 50 mg/m² body surface to 180 mg/m² body surface, especially preferably of from 50 mg/m² body surface to 150 mg/m² body surface of the treated human subject.

10. Method for producing an active substance delivery system, preferably according to one of the preceding claims, or nanoparticles as defined in any one of the preceding claims, comprising or consisting of the following steps
i) providing PLGA and dissolving PLGA in a solvent,
ii) providing one, two, three or more active substances and dissolving the one, two, three or more active substances in a solvent,
iii) mixing the solutions obtained in step i) and ii),
iv) providing a polyvinyl alcohol (PVA) solution, preferably a solution containing 0.1 to 5 wt.-%, particularly preferably 0.2 to 3 wt.-% of PVA, based on the total amount of the solution,
v) adding the mixture obtained in step iii) to the solution obtained in step iv) to produce nanoparticles,
vi) optionally: evaporating the solvent, one of or the solvents used in step i) and ii),
vii) purifying the nanoparticles and subsequently dispersing the nanoparticles in a dispersant, preferably in a pharmaceutically acceptable solvent,
viii) providing one, two, three or more surface coating material(s) and coating the nanoparticles therewith, and
ix) optionally: providing and adding one or more further pharmaceutically acceptable components to the nanoparticles
wherein in step v) the amounts of the mixture and of the solution are selected such that a ratio of a least 1 ml of solution per 5 mg of the summed weight of PLGA and the active substance(s) is achieved, and
wherein the addition in step v) is performed by injection, preferably constant injection, preferably at a rate in a range of from 0.1 to 1.75, preferably in a range of from 0.25 to 1.5 ml/min, particularly preferably at a rate in a range of from 0.35 to 1.25 ml/min.

11. Method according to claim 10, wherein the solvent used in step i) and/or the solvent used in step ii) comprises or consists of one or more substances selected from the group consisting of acetone ethyl acetate, chlorinated solvents, DMSO, methylated solvents, tetrahydrofuran, halogenated hydrocarbons such as chloroform and dichloromethane, dioxanes, acetonitrile,
and/or
wherein the solvent used in step iv) for providing a PVA solution comprises or consists of one or more substances selected from the group consisting of water, ethanol and saline,
and/or
wherein the dispersant used in step vii) comprises or consists of one or more substances selected from the group consisting of saline, glucose, surfactants, pH modifiers and water.

12. Method according to one of claims 10 or 11, wherein the, one or all active substance(s) is/are (a)
i) chemotherapeutic agent(s), preferably selected from the group consisting of chemotherapeutic agents used against a cancer type selected from the group consisting of gastrointestinal cancer, gynaecological cancer, prostate cancer, peritoneal metastasis, leukaemia, lymphoma, soft-tissue sarcoma, multiple myeloma, breast cancer, bladder cancer, lung cancer, thyroid cancer, Kaposi's sarcoma, particularly preferably ovarian cancer, peritoneal metastasis colorectal cancer and stomach cancer, particularly preferably selected from the group consisting of paclitaxel, cisplatin, oxaliplatin and doxorubicin,
and/or
ii) immunomodulatory drug(s), preferably selected from the group consisting of curcumin, lutein, hesperidin, apigenin, hesperetin, ajoene, arctigenin, β-carotene, epigallocatechin-3-gallate, glabridin or quinic acid.

13. Method according to one of claims 10 to 12, wherein the one or further pharmaceutically acceptable components is/are selected from the group consisting of carriers, polymers, surfactants, stabilizers, wetting agents, emulsifiers, antioxidants, pH influencing agents, disintegrants, recrystallization agents, fluxing agents, preservatives, solvents, salts fillers, binders, foamers, defoamers, lubricants, adsorbents for adjusting the osmotic pressure and buffers.
